# EUROPEAN PATENT APPLICATION

(11) **EP 4 628 038 A1**
(43) Date of publication of application: **08.10.2025**
(21) Application number: 23908830.5
(22) Date of filing: 09.03.2023
(51) Int. Cl.: A61B 18/12, A61B 18/14, A61N 1/18, A61N 1/06, A61M 37/00

(54) **RADIO FREQUENCY ARRAY CONTROL APPARATUS AND RADIO FREQUENCY THERAPEUTIC INSTRUMENT**

(30) Priority: 30.12.2022 CN 202211726766
(71) Applicant: Shenzhen Peninsula Medical Group, Shenzhen, Guangdong 518000 (CN)
(72) Inventor: LIANG, Yongsheng, Shenzhen, Guangdong 518000 (CN); LI, Yanan, Shenzhen, Guangdong 518000 (CN); LEI, Xiaobing, Shenzhen, Guangdong 518000 (CN); DING, Yi, Shenzhen, Guangdong 518000 (CN)
(74) Representative: Osterhoff, Utz
(86) International application number: PCT/CN2023/080490
(87) International publication number: WO 2024/138873

(57) **Abstract**

Disclosed are a radio frequency (RF) array control device and a RF treatment instrument. The device includes: a mode determination module, configured to determine a target output mode according to received instruction information; an electrode group determination module, configured to determine at least one target electrode group from multiple candidate electrodes of the RF electrode array according to the target output mode; a control module configured to control the RF power supply to output RF energy to the target electrode group according to the preset treatment duration so that the target electrode group transmits the RF energy to the target area; and an electrode group update module configured to skip the target electrode group from multiple candidate electrodes and to obtain updated multiple candidate electrode groups. The control module is also configured to return to perform a step of determining at least one target electrode group from the plurality of candidate electrodes in the RF electrode array according to the target output mode until there is no candidate electrode.

## Description

### CROSS-REFERENCE TO RELATED APPLICATIONS

The present application claims priority to Chinese Patent Application No. 202211726766.9, entitled "RADIO FREQUENCY ARRAY CONTROL DEVICE AND RADIO FREQUENCY THERAPEUTIC INSTRUMENT", filed on December 30, 2022, and the entire contents of which are incorporated herein by reference.

### TECHNICAL FIELD

The present application relates to the technical field of medical devices, and in particular to a radio frequency array control device and a radio frequency treatment instrument having the same.

### BACKGROUND

In the related art, radio frequency (RF) arrays can be used in skin tightening and wrinkle removal, scar smoothing, pore tightening, skin rejuvenation and whitening, and acne scar treatment. The RF arrays include RF microneedle arrays, non-invasive RF arrays, etc. In the actual application of RF arrays, in order to achieve the desired treatment effect, the RF energy output by the RF power supply needs to be controlled to be within a certain RF output power range and the preset treatment duration, and the RF energy output from the RF power supply needs to be controlled to act on the target area.

### SUMMARY

Currently, it is not possible to accurately control the radio frequency (RF) power supply to output RF energy.

The main purpose of the present application is to provide a RF array control device and a RF treatment instrument, aiming to solve the technical problem of currently unable to accurately control the RF power supply to output RF energy.

In order to achieve the above purpose, the present application provides a RF array control device, applied to a RF treatment instrument, the RF treatment instrument includes:
the RF power supply, configured to output the RF energy; and
the RF electrode array, configured to be connected to the RF power supply.

The device includes:
a mode determination module, configured to determine a target output mode according to received instruction information;
an electrode group determination module, configured to determine at least one target electrode group from a plurality of candidate electrodes in a RF electrode array according to the target output mode;
a control module, configured to control a RF power supply to output RF energy to the target electrode group according to a preset treatment duration, so that the target electrode group transmits the RF energy to a target area; and
an electrode group update module, configured to skip the target electrode group from the plurality of candidate electrodes and obtain the updated plurality of candidate electrode groups.

The control module is also configured to return to perform a step of determining at least one target electrode group from the plurality of candidate electrodes in the RF electrode array according to the target output mode until there is no candidate electrode.

In an embodiment of the present application, the RF treatment instrument also includes a negative plate detachably connected to the RF power supply;
the target output mode includes a unipolar mode;
the electrode group determination module includes:
   a first electrode group determination unit configured to determine one target electrode group from the plurality of candidate electrodes in the RF electrode array according to the unipolar mode; and
   a first connection control unit configured to control the one target electrode group to be connected to one end of the RF power supply, and control the other end of the RF power supply to be connected to the negative plate.

In an embodiment of the present application, the first electrode group determination unit is further configured to determine at least one candidate electrode group from the plurality of candidate electrodes of the RF electrode array and determine the one target electrode group from the at least one candidate electrode group, according to the unipolar mode.

In an embodiment of the present application, the target output mode includes a bipolar mode;
the electrode group determination module includes:
a second electrode group determination unit, configured to determine two target electrode groups from the plurality of candidate electrodes of the RF electrode array according to the bipolar mode; and
a first connection control unit configured to control one of the two target electrode groups to be connected to a first output end of the RF power supply, and the other to be connected to a second output end of the RF power supply.

In an embodiment of the present application, the plurality of candidate electrodes of the RF electrode array include at least one candidate first polarity electrode group and at least one candidate second polarity electrode group, and the at least one candidate first polarity electrode group and the at least one candidate second polarity electrode group are arranged alternately in sequence; the second electrode group determination unit is further configured to determine a first polarity target electrode group from the at least one candidate first polarity electrode group in the RF electrode array and determine a second polarity target electrode group from the at least one candidate second polarity electrode group, according to the bipolar mode, and the first polarity target electrode group and the second polarity target electrode group are adjacent to each other; and the first connection control unit is further configured to control the first polarity target electrode group to be connected to the first output end of the RF power supply, and control the second polarity target electrode group to be connected to the second output end of the RF power supply.

In an embodiment of the present application, the plurality of candidate electrodes of the RF electrode array include at least one candidate first polarity electrode group and at least one candidate second polarity electrode group, and the at least one candidate first polarity electrode group and the at least one candidate second polarity electrode group are arranged alternately in sequence; the second electrode group determination unit is further configured to determine a first polarity target electrode group from the at least one candidate first polarity electrode group in the RF electrode array and determine a second polarity target electrode group from the at least one candidate second polarity electrode group, according to the bipolar mode; and the first connection control unit is further configured to control the first polarity target electrode group to be connected to the first output end of the RF power supply, and control the second polarity target electrode group to be connected to the second output end of the RF power supply.

In an embodiment of the present application, the plurality of candidate electrodes of the RF electrode array include at least one candidate first polarity electrode and at least one candidate second polarity electrode, and the at least one candidate first polarity electrode and the at least one candidate second polarity electrode are arranged alternately in sequence; the second electrode group determination unit is further configured to determine a first polarity target electrode group from the at least one candidate first polarity electrode and determine a second polarity target electrode group from the at least one candidate second polarity electrode, according to the bipolar mode, and the first polarity target electrode group and the second polarity target electrode group are adjacent to each other; and the first connection control unit is further configured to control the first polarity target electrode group to be connected to the first output end of the RF power supply, and control the second polarity target electrode group to be connected to the second output end of the RF power supply.

In an embodiment of the present application, the plurality of candidate electrodes of the RF electrode array include at least one candidate first polarity electrode and at least one candidate second polarity electrode, and the at least one candidate first polarity electrode and the at least one candidate second polarity electrode are arranged alternately in sequence; the second electrode group determination unit is further configured to determine a first polarity target electrode group from the at least one candidate first polarity electrode and determine a second polarity target electrode group from the at least one candidate second polarity electrode, according to the bipolar mode; and the first connection control unit is further configured to control the first polarity target electrode group to be connected to the first output end of the RF power supply, and control the second polarity target electrode group to be connected to the second output end of the RF power supply.

In an embodiment of the present application, the preset treatment duration T is in a range of 1ms to 50ms.

In an embodiment of the present application, the preset treatment duration T is further in a range of 1ms to 30ms.

In an embodiment of the present application, the control module is further configured to control the RF power supply to sequentially output the RF energy to the target electrode group according to the preset treatment duration and the preset RF output power range, so that the target electrode group transmits the RF energy to the target area; and the preset RF output power range P is in a range of 1W to 25W.

In an embodiment of the present application, the preset RF output power range P is further in a range of 1W to 15W.

In an embodiment of the present application, the number of electrodes in each of the candidate electrode groups is N in a range of 1 to 25; the number of electrodes in each of the candidate electrode groups is the same; or any two of the candidate electrode groups have the numbers of electrodes differing no more than two.

In an embodiment of the present application, the number of electrodes in each of the candidate electrode groups is N in a range of 1 to 6.

The present application also proposes the RF treatment instrument, which includes:
a RF power supply, configured to output RF energy;
a RF electrode array, connected to the RF power supply; and
the RF array control device as described above.

The embodiment of the present application proposes a RF array control device and a RF treatment instrument. During the treatment process of the RF treatment instrument in the present application, only a number of target electrode groups corresponding to the target output modes are outputting energy within each preset treatment duration. Then, within the next preset treatment duration, a new target electrode group is selected from unused electrodes for treatment until the entire RF electrode array is traversed. Compared to the related art that the RF energy lasts long enough in the target area, the present application groups the electrodes of the RF electrode array and accurately delivers the RF energy to the tissue corresponding to each electrode in turn, to achieve the original effect with a shorter action time, reduce pain, and improve the user's experience.

### BRIEF DESCRIPTION OF THE DRAWINGS

FIG. 1 is a schematic structural diagram of a radio frequency array control device according to an embodiment of the present application.
FIG. 2 is a schematic structural diagram of a radio frequency treatment instrument provided by the present application.

The realization of the purpose, functional features and advantages of the present application will be further described in conjunction with the embodiments, with reference to the accompanying drawings.

### DETAILED DESCRIPTION OF THE EMBODIMENTS

It should be understood that the specific embodiments described here are only used to explain the present application and are not used to limit the present application.

In the related art, the arrays of electrodes in radio frequency (RF) technology can be used in skin tightening and wrinkle removal, scar smoothing, pore tightening, skin rejuvenation and whitening, and acne scar treatment. The RF arrays include RF microneedle arrays, non-invasive RF arrays, etc. In the actual application of RF arrays, in order to achieve the desired treatment effect, the RF energy output by the RF power supply needs to be controlled to be within a certain RF output power range and continue for the preset treatment duration, and the RF energy output from the RF power supply needs to be controlled to act on the target area.

However, it is currently not possible to accurately control the RF power supply to output RF energy.

The present application provides a solution. The embodiments of the present application propose a RF array control device and a RF treatment instrument. During the treatment process of the RF treatment instrument in the present application, only a number of target electrode groups corresponding to the target output modes are outputting energy within each preset treatment duration. Then, within the next preset treatment duration, a new target electrode group is selected from unused electrodes for treatment until the entire RF electrode array is traversed. Compared to the related art that the RF energy lasts long enough in the target area, the present application groups the electrodes of the RF electrode array and accurately delivers the RF energy to the tissue corresponding to each electrode in turn, to achieve the original effect with a shorter action time, reduce pain, and improve the user's experience.

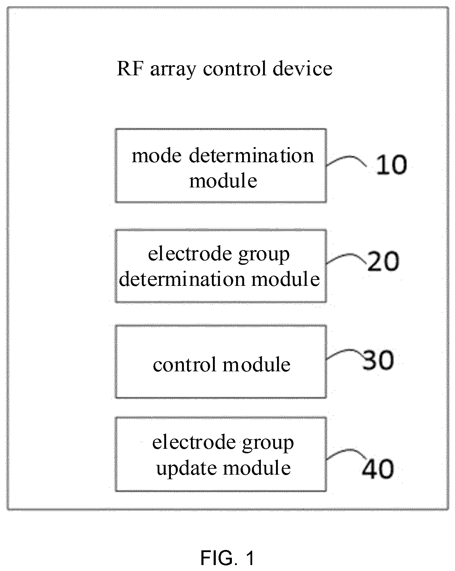

The present application provides an embodiment of a RF array control device. Referring to FIG. 1, FIG. 1 shows a schematic structural diagram of the RF array control device according to an embodiment of the present application.

It should be noted that although a logical order is shown in the flowchart, in some cases, steps shown or described can be performed in a different order than here.

In this embodiment, the RF array control device is used in the RF treatment instrument. The RF treatment instrument includes a RF power supply 100 and a RF electrode array 200.

The RF power supply 100 is configured to output RF energy.

The RF electrode array 200 is connected to the RF power supply 100.

It should be understood that the RF power supply 100 can be a continuous output power supply or a pulse output power supply or a continuous and pulse output power supply. The RF power supply 100 can include only one, that is, a single RF power supply 100 supplies power to the RF electrode array 200. The RF electrode array is provided on a treatment end of a handle of the RF treatment instrument. Alternatively, the RF power supply 100 can also include a plurality of RF power supplies 100. The plurality of RF power supplies 100 are respectively connected to the switch module of the RF treatment instrument. The switch module is controlled by the control unit of the RF treatment instrument and is used to realize the connection and disconnection between the RF electrode array and the RF power supply.

The RF electrode array 200 includes a series of electrodes that are mutually coupled and tiny and have array cross-sections arranged according to certain rules. The user groups the electrodes in the RF electrode array in advance to obtain multiple electrode groups. Each electrode group can include one electrode or multiple electrodes. When there are multiple electrodes in the same group in the electrode group, the electrodes are connected in parallel. It can be understood that at the beginning of treatment, all electrode groups are available for selection, that is, all are the electrode groups to be selected.

It should be noted that the switch module can be used to realize connection and disconnection between the RF electrode array 200 and the RF power supply 100. If the switch module is in an open state, the target electrode group in the RF electrode array 200 is connected to the RF power supply 100; if the switch module is in a closed state, the target electrode group in the RF electrode array 200 is disconnected from the RF power supply 100.

In this embodiment, the device includes a mode determination module 10, a electrode group determination module 20, a control module 30 and a electrode group update module 40.

The mode determination module 10 is used to determine the target output mode according to the received instruction information.

It should be understood that the instruction information can be input by the user through corresponding buttons on the RF treatment instrument or an external device such as a terminal device connected to the RF treatment instrument and then sent to the RF array control device. The instruction information includes mode choosing information, which can be chose by the user in unipolar mode or bipolar mode. It can be understood that the instruction information includes one or more than modes choosing information.

If the instruction information sent by the user is of the unipolar mode, the RF array control device determines the target output mode to be the unipolar mode according to the received instruction information; if the instruction information sent by the user is of the bipolar mode, the RF array control device determines the target output mode to be the bipolar mode according to the received instruction information.

The electrode group determination module 20 is configured to determine at least one target electrode group from a plurality of candidate electrodes in the RF electrode array according to the target output mode.

It should be understood that the target electrode group is the target object to which the RF power supply 100 outputs RF energy. If the target output mode is the unipolar mode, since the negative plate is connected to the RF power supply at this time, the RF array control device determines one target electrode group from the plurality of candidate electrodes in the RF electrode array and provides positive polarity. If the target output mode is the bipolar mode, the RF array control device determines two target electrode groups from the plurality of candidate electrodes in the RF electrode array, and the two target electrode groups provide positive polarity and negative polarity respectively.

The control module 30 is configured to control the RF power supply to output RF energy to the target electrode group according to the preset treatment duration, so that the target electrode group transmits the RF energy to the target area.

It should be understood that the preset treatment duration can be configured in advance by the manufacturer, or can be set by the user and included in the instruction information.

The RF array control device controls the RF power supply to output RF energy, and does not stop outputting RF energy to the target electrode group until the output time reaches the preset treatment time.

The electrode group update module 40 is configured to skip the target electrode group from the plurality of candidate electrodes and obtain the updated plurality of candidate electrode groups.

The control module 30 is also configured to return to perform the step of determining at least one target electrode group from the plurality of candidate electrodes in the RF electrode array according to the target output mode until there is no candidate electrode.

It should be understood that after the target electrode group transmits RF energy to the target area, in order to ensure that the tissues in the target area corresponding to the RF electrode array can be treated, after the treatment of each target electrode group is completed, the electrode group update module of the RF array control device skips the target electrode group that has output RF energy from the plurality of candidate electrodes in the RF electrode array, and obtains the updated plurality of candidate electrode groups. Then, within the next preset treatment duration, a new target electrode group is selected for treatment from the unused electrodes, that is, from the plurality of candidate electrodes at this time. During the treatment process, the electrode group determination module 20, the control module 30 and the electrode group update module 40 repeatedly execute corresponding steps until all the electrodes in the RF electrode array have been used in this treatment course, so that there are no unused candidate electrodes, that is, there is no candidate electrode.

It is easy to understand that in order to achieve the expected treatment effect, the existing RF treatment instrument needs to control the RF energy output by the RF power supply to be within a certain RF output power range, and the energy output needs to continue for a preset treatment time.

In this embodiment, during the treatment process of the RF treatment instrument, only a number of target electrode groups corresponding to the target output mode are outputting energy within each preset treatment duration. Then, in the next preset treatment duration, a new target electrode group is selected from unused electrodes for treatment until the entire RF electrode array is traversed. This embodiment groups the RF electrode array and accurately delivers the RF energy to the tissue corresponding to each electrode in turn, to achieve the original effect with a shorter action time, reduce pain, and improve the user's experience.

In addition, it should be understood that during the treatment process of traditional RF treatment instrument, all electrode groups are connected to the RF power supply to output RF energy. However, due to the different impedance between tissues, there is an energy output path between all electrode groups, that is, the energy output of the RF treatment instrument is uneven, which can easily lead to local energy accumulation and overheating, affecting the treatment effect. In this embodiment, therefore, the control module 30 of the RF array control device is also used to return to perform the step of determining at least one target electrode group from a plurality of candidate electrodes in the RF electrode array according to the target output mode, until there is no candidate electrode, which improves the uniformity of RF energy distribution in the entire target area, shortens the treatment time and improves efficacy.

As a specific implementation, the RF treatment instrument also includes a negative plate, and the negative plate is detachably connected to the RF power supply.

If the target output mode is the unipolar mode, the negative plate can be connected to the RF power supply, so the number of target electrode groups is one. At this time, the RF array control device determines one target electrode group from the multiple candidate electrodes in the RF electrode array. The negative plate 300 can be used to form a conductive circuit for RF energy.

The target output mode includes the unipolar mode.

The electrode group determination module 20 includes a first electrode group determination unit 201 and a first connection control unit 202.

The first electrode group determination unit 201 is configured to determine one target electrode group from a plurality of candidate electrodes in the RF electrode array according to the unipolar mode.

The first connection control unit 202 is configured to control the one target electrode group to be connected to one end of the RF power supply, and control the other end of the RF power supply to be connected to the negative plate.

It should be understood that if the target output mode is the unipolar mode, the negative plate is connected to the RF power supply, and the RF array control device determines one target electrode group from multiple candidate electrodes in the RF electrode array. If the target output mode is the unipolar mode, the target electrode group is connected to one end of the RF power supply, and the other end of the RF power supply is connected to the negative plate 300. The negative plate 300 can be used to form a conductive circuit of RF energy.

In this embodiment, by connecting one target electrode group to one end of the RF power supply in the unipolar mode, and controlling the other end of the RF power supply to be connected to the negative plate, it is beneficial to form a conductive circuit of RF energy.

Further, in a specific embodiment, the first electrode group determination unit 201 is also configured to determine at least one candidate electrode group from a plurality of candidate electrodes in the RF electrode array according to the unipolar mode, and determine the target electrode group from the at least one candidate electrode group.

It should be understood that in this embodiment, multiple candidate electrode groups are divided from multiple candidate electrodes of the RF electrode array. Further, the number of electrodes in each of candidate electrode groups is N in a range of 1 to 25; and the number of electrodes in each group of candidate electrode groups is the same; or any two of candidate electrode groups have the numbers of electrodes differing no more than two. Further, the number of electrodes in each of the candidate electrode group is N in a range of 1 to 6. The first electrode group determination unit 201 determines one target electrode group from the divided multiple candidate electrode groups.

In this embodiment, after grouping the multiple candidate electrodes, one target electrode group is selected from the multiple candidate electrode groups, which improves the convenience of user operation.

As a specific implementation, the target output mode includes a bipolar mode.

In addition, if the target output mode is the bipolar mode and the negative plate is disconnected from the RF power supply, the number of target electrode groups is two. At this time, the RF array control device determines two target electrode groups from the plurality of candidate electrodes in the RF electrode array.

The electrode group determination module 20 includes a second electrode group determination unit 203 and the first connection control unit 202.

The second electrode group determination unit 203 is configured to determine two target electrode groups from a plurality of candidate electrodes in the RF electrode array according to the bipolar mode.

The first connection control unit 202 is configured to control one of the two target electrode groups to be connected to a first output end of the RF power supply, and the other to be connected to a second output end of the RF power supply.

It should be understood that if the target output mode is the bipolar mode, the negative plate is disconnected from the RF power supply, and the RF array control device determines two target electrode groups from a plurality of candidate electrodes in the RF electrode array. The first connection control unit can control one of the two target electrode groups to be connected to the first output end of the RF power supply through the switch unit, and the other to be connected to the second output end of the RF power supply, which is beneficial to control the RF power supply to simultaneously output RF energy to the two target electrode groups, so that the two target electrode groups transmit the RF energy to the target area to achieve the expected effect.

As a specific implementation, the multiple candidate electrodes of the RF electrode array include at least one candidate first polarity electrode group and at least one candidate second polarity electrode group, at least one candidate first polarity electrode group and at least one candidate second polarity electrode group are arranged alternately in sequence.

The second electrode group determination unit 203 is further configured to determine a first polarity target electrode group from at least one candidate first polarity electrode group in the RF electrode array according to the bipolar mode, and determine a second polarity target electrode group from at least one candidate second polarity electrode group, and the first polarity target electrode group and the second polarity target electrode group are adjacent to each other.

The first connection control unit 202 is also configured to control the connection between the first polarity target electrode group and the first output end of the RF power supply, and control the connection between the second polarity target electrode group and the second output end of the RF power supply.

It should be understood that the polarity of the first polarity electrode group is different from the polarity of the second polarity electrode group. If the polarity of the first polarity electrode group is positive, the polarity of the second polarity electrode group is negative; if the polarity of the second polarity electrode group is positive, the polarity of the first polarity electrode group is negative. Since in the RF electrode array, at least one first polarity electrode group and at least one second polarity electrode group are arranged alternately in sequence, two adjacent target electrode groups determined from the plurality of electrodes in the RF electrode array necessarily include one first polarity electrode group and one second polarity electrode group.

In this embodiment, in the RF electrode array, after a plurality of candidate first polarity electrode groups and a plurality of candidate second polarity electrode groups are divided from the plurality of candidate electrodes in the RF electrode array, one first polarity target electrode group and one second polarity target electrode group adjacent to each other are determined from a plurality of candidate first polarity electrode groups and a plurality of candidate second polarity electrode groups, so that in the bipolar mode, treatment is only performed on the target area corresponding to the first polarity target electrode group and the second polarity target electrode group adjacent to each other, which is beneficial for the uniformity of RF energy distribution in the entire target area.

As a specific implementation, the multiple candidate electrodes of the RF electrode array include at least one candidate first polarity electrode group and at least one candidate second polarity electrode group, and at least one candidate first polarity electrode group and at least one second polarity electrode group are arranged alternately in sequence.

The second electrode group determination unit 203 is also configured to determine a first polarity target electrode group from at least one candidate first polarity electrode group in the RF electrode array and determine a second polarity target electrode group from at least one candidate second polarity electrode group, according to the bipolar mode.

The first connection control unit 202 is also configured to control the connection between the first polarity target electrode group and the first output end of the RF power supply, and control the connection between the second polarity target electrode group and the second output end of the RF power supply.

It should be understood that in this embodiment, a plurality of candidate first polarity electrode groups and a plurality of candidate second polarity electrode groups are divided from a plurality of candidate electrodes in the RF electrode array. The first electrode group determination unit 201 arbitrarily determines one first polarity target electrode group and one second polarity target electrode group from a plurality of candidate first polarity electrode groups and a plurality of candidate second polarity electrode groups. The first connection control unit 202 controls the first polarity target electrode group to be connected to the first output end of the RF power supply, and controls the second polarity target electrode group to be connected to the second output end of the RF power supply.

In this embodiment, in the RF electrode array, after a plurality of candidate first polarity electrode groups and a plurality of candidate second polarity electrode groups are divided from the plurality of candidate electrodes in the RF electrode array, one first polarity target electrode group and one second polarity target electrode group are arbitrarily determined from a plurality of candidate first polarity electrode groups and a plurality of candidate second polarity electrode groups, so that in the bipolar mode, treatment is only performed on the target area corresponding to the first polarity target electrode group and the second polarity target electrode group, which is beneficial for the uniformity of RF energy distribution in the entire target area, and improves the convenience of user operation.

As a specific implementation, the plurality of candidate electrodes of the RF electrode array include at least one candidate first polarity electrode and at least one candidate second polarity electrode, and at least one candidate first polarity electrode and at least one candidate second polarity electrode are arranged alternately in sequence.

The second electrode group determination unit 203 is also configured to determine a first polarity target electrode group from at least one candidate first polarity electrode and determine a second polarity target electrode group from at least one candidate second polarity electrode, according to the bipolar mode. The first polarity target electrode group and the second polarity target electrode group are adjacent to each other.

The first connection control unit 202 is also configured to control the connection between the first polarity target electrode group and the first output end of the RF power supply, and control the connection between the second polarity target electrode group and the second output end of the RF power supply.

It should be understood that in this embodiment, a plurality of candidate first polarity electrodes and a plurality of candidate second polarity electrodes are arranged alternately in sequence. The second electrode group determination unit 203 directly selects the first polarity target electrode group from the plurality of candidate first polarity electrodes, and select the second polarity target electrode group from the plurality of candidate second polarity electrodes. The first polarity target electrode group and the second polarity target electrode group are adjacent to each other. The first connection control unit 202 controls the first polarity target electrode group to be connected to the first output end of the RF power supply, and controls the second polarity target electrode group to be connected to the second output end of the RF power supply.

In this embodiment, in the candidate first polarity electrodes and the candidate second polarity electrodes of the RF array, the user can determine the first polarity target electrode group from the plurality of candidate first polarity electrodes, and determine the second polarity target electrode group from the plurality of candidate second polarity electrodes. The first polarity target electrode group and the second polarity target electrode group are adjacent to each other, which can improve the user's selectivity, and achieve the same effect with lower output power and shorter energy release time.

As a specific implementation, the plurality of candidate electrodes of the RF electrode array include at least one candidate first polarity electrode and at least one candidate second polarity electrode, and at least one candidate first polarity electrode and at least one candidate second polarity electrode are arranged alternately in sequence.

The second electrode group determination unit is further configured to determine the first polarity target electrode group from at least one candidate first polarity electrodes and determine the second polarity target electrode group from at least one candidate second polarity electrodes, according to the bipolar mode.

The first connection control unit is also configured to control the connection between the first polarity target electrode group and the first output end of the RF power supply, and control the connection between the second polarity target electrode group and the second output end of the RF power supply.

It should be understood that the plurality of candidate first polarity electrodes and the plurality of candidate second polarity electrodes are arranged alternately in sequence. The second electrode group determination unit 203 directly selects the first polarity target electrode group from the plurality of candidate first polarity electrodes, and selects the second polarity target electrode group from the plurality of candidate second polarity electrodes. The first connection control unit 202 controls the first polarity target electrode group to be connected to the first output end of the RF power supply, and controls the second polarity target electrode group to be connected to the second output end of the RF power supply.

In this embodiment, in the candidate first polarity electrodes and the candidate second polarity electrodes of the RF array, the user can determine the first polarity target electrode group from the plurality of candidate first polarity electrodes, and determine the second polarity target electrode group from the plurality of candidate second polarity electrodes, which can improve the user's selectivity, and achieve the same effect with lower output power and shorter energy release time.

As a specific implementation, the preset treatment duration T is in a range of 1ms to 50ms.

As a preferred implementation, in order to further reduce the pain during the energy release process and improve the comfort of the device experience, the energy release time of each target electrode group can be further shortened. At this time, the preset treatment duration T is further in a range of 1ms to 30ms.

As a specific implementation, the control module is also configured to control the RF power supply to sequentially output the RF energy to the target electrode group according to the preset treatment duration and the preset RF output power range, so that the target electrode group transmits the RF energy to the target area.

The preset RF output power range P is in a range of 1W to 25W.

As a preferred embodiment, the preset RF output power range P is further in a range of 1W to 15W.

It should be understood that in order to further reduce the pain during the energy release process and improve the comfort of the device experience, the energy release power of each target electrode group can be further reduced, that is, the same effect can be achieved with lower output power.

In this embodiment, by limiting the preset treatment duration and the preset RF output power range to a certain range, it is beneficial to control the treatment duration and output power of the RF energy output by the RF power supply 100, thereby avoid increasing user pain due to excessive long treatment duration or excessive high RF output power.

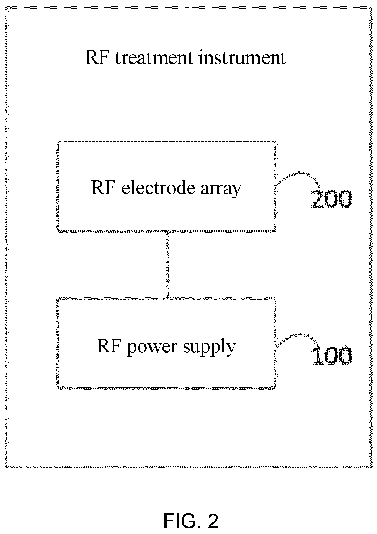

Based on the same concept, the present application also provides a RF treatment instrument. Referring to FIG. 2, which is a schematic structural diagram of a RF treatment instrument provided by the present application. The RF treatment instrument includes a RF power supply 100 configured to output RF energy, a RF electrode array 200 connected to the RF power supply and the RF array control device as described above.

In this embodiment, during the treatment process of the RF treatment instrument, only a number of target electrode groups corresponding to the target output modes are outputting energy within each preset treatment duration. Then, within the next preset treatment duration, a new target electrode group is selected from unused electrodes for treatment until the entire RF electrode array is traversed. Compared to the related art that the RF energy lasts long enough in the target area, the present application divides the electrodes of the RF electrode array into groups and accurately delivers the RF energy to the tissue corresponding to each electrode in turn, to achieve the original effect with a shorter action time, reduce pain, and improve the user's experience.

The above are only some embodiments of the present application, and are not intended to limit the scope of the present application. Any equivalent structures or equivalent process transformations made using the contents of the description and the accompanying drawings of the present application, or direct or indirect applications in other related technical fields, are all included in the scope of the present application.

## Claims

1. A radio frequency (RF) array control device, applied to a RF treatment instrument, **characterized in that** the control device comprises:
a mode determination module, configured to determine a target output mode according to a received instruction information;
an electrode group determination module, configured to determine at least one target electrode group from a plurality of candidate electrodes in a RF electrode array according to a target output mode;
a control module, configured to control a RF power supply to output RF energy to the target electrode group according to a preset treatment duration, so that the target electrode group transmits the RF energy to a target area, wherein the control module is also configured to return to perform a step of determining at least one target electrode group from the plurality of candidate electrodes in the RF electrode array according to the target output mode until there is no candidate electrode; and
an electrode group update module, configured to skip the target electrode group from the plurality of candidate electrodes and obtain the updated plurality of candidate electrode groups,
wherein the RF treatment instrument comprises:
the RF power supply, configured to output the RF energy; and
the RF electrode array, configured to be connected to the RF power supply.

2. The RF array control device according to claim 1, wherein the RF treatment instrument further comprises a negative plate detachably connected to the RF power supply;
the target output mode comprises a unipolar mode;
the electrode group determination module comprises:
a first electrode group determination unit, configured to determine one target electrode group from the plurality of candidate electrodes in the RF electrode array according to the unipolar mode; and
a first connection control unit, configured to control the one target electrode group to be connected to one end of the RF power supply, and control the other end of the RF power supply to be connected to the negative plate.

3. The RF array control device according to claim 2, wherein the first electrode group determination unit is further configured to determine at least one candidate electrode group from the plurality of candidate electrodes of the RF electrode array and determine the one target electrode group from the at least one candidate electrode group, according to the unipolar mode.

4. The RF array control device according to claim 1, wherein the target output mode comprises a bipolar mode;
the electrode group determination module comprises:
a second electrode group determination unit, configured to determine two target electrode groups from the plurality of candidate electrodes of the RF electrode array according to the bipolar mode; and
a first connection control unit, configured to control one of the two target electrode groups to be connected to a first output end of the RF power supply, and the other to be connected to a second output end of the RF power supply.

5. The RF array control device according to claim 4, wherein the plurality of candidate electrodes of the RF electrode array comprise at least one candidate first polarity electrode group and at least one candidate second polarity electrode group, and the at least one candidate first polarity electrode group and the at least one candidate second polarity electrode group are arranged alternately in sequence;
the second electrode group determination unit is further configured to determine a first polarity target electrode group from the at least one candidate first polarity electrode group in the RF electrode array and determine a second polarity target electrode group from the at least one candidate second polarity electrode group, according to the bipolar mode, and the first polarity target electrode group and the second polarity target electrode group are adjacent to each other; and
the first connection control unit is further configured to control the first polarity target electrode group to be connected to the first output end of the RF power supply, and control the second polarity target electrode group to be connected to the second output end of the RF power supply.

6. The RF array control device according to claim 4, wherein the plurality of candidate electrodes of the RF electrode array comprise at least one candidate first polarity electrode group and at least one candidate second polarity electrode group, and the at least one candidate first polarity electrode group and the at least one candidate second polarity electrode group are arranged alternately in sequence;
the second electrode group determination unit is further configured to determine a first polarity target electrode group from the at least one candidate first polarity electrode group in the RF electrode array and determine a second polarity target electrode group from the at least one candidate second polarity electrode group, according to the bipolar mode; and
the first connection control unit is further configured to control the first polarity target electrode group to be connected to the first output end of the RF power supply, and control the second polarity target electrode group to be connected to the second output end of the RF power supply.

7. The RF array control device according to claim 4, wherein the plurality of candidate electrodes of the RF electrode array comprise at least one candidate first polarity electrode and at least one candidate second polarity electrode, and the at least one candidate first polarity electrode and the at least one candidate second polarity electrode are arranged alternately in sequence;
the second electrode group determination unit is further configured to determine a first polarity target electrode group from the at least one candidate first polarity electrode and determine a second polarity target electrode group from the at least one candidate second polarity electrode, according to the bipolar mode, and the first polarity target electrode group and the second polarity target electrode group are adjacent to each other; and
the first connection control unit is further configured to control the first polarity target electrode group to be connected to the first output end of the RF power supply, and control the second polarity target electrode group to be connected to the second output end of the RF power supply.

8. The RF array control device according to claim 4, wherein the plurality of candidate electrodes of the RF electrode array comprise at least one candidate first polarity electrode and at least one candidate second polarity electrode, and the at least one candidate first polarity electrode and the at least one candidate second polarity electrode are arranged alternately in sequence;
the second electrode group determination unit is further configured to determine a first polarity target electrode group from the at least one candidate first polarity electrode and determine a second polarity target electrode group from the at least one candidate second polarity electrode, according to the bipolar mode; and
the first connection control unit is further configured to control the first polarity target electrode group to be connected to the first output end of the RF power supply, and control the second polarity target electrode group to be connected to the second output end of the RF power supply.

9. The RF array control device according to claim 1, wherein the preset treatment duration T is in a range of 1ms to 50ms.

10. The RF array control device according to claim 9, wherein the preset treatment duration T is further in a range of 1ms to 30ms.

11. The RF array control device according to any one of claims 1 to 8, wherein the control module is further configured to control the RF power supply to sequentially output the RF energy to the target electrode group according to the preset treatment duration and a preset RF output power range, so that the target electrode group transmits the RF energy to the target area; and the preset RF output power range P is in a range of 1W to 25W.

12. The RF array control device according to claim 11, wherein the preset RF output power range P is further in a range of 1W to 15W.

13. The RF array control device according to claim 1, wherein the number of electrodes in each of the candidate electrode groups is N in a range of 1 to 25;
the number of electrodes in each of the candidate electrode groups is the same; or
any two of the candidate electrode groups have the numbers of electrodes differing no more than two.

14. The RF array control device according to claim 1, wherein the number of electrodes in each of the candidate electrode groups is N in a range of 1 to 6.

15. A radio frequency (RF) treatment instrument, **characterized by** comprising:
a RF power supply, configured to output RF energy;
a RF electrode array, connected to the RF power supply; and
the RF array control device according to any one of claims 1 to 14.
